# EUROPEAN PATENT APPLICATION

(11) **EP 3 466 469 A1**
(43) Date of publication of application: **10.04.2019**
(21) Application number: 18198165.5
(22) Date of filing: 02.10.2018
(51) Int. Cl.: A61M 11/02, A61M 11/00

(54) **INHALATION DEVICE FOR GENERATING AEROSOL AND METHOD FOR GENERATING AEROSOL**

(30) Priority: 04.10.2017 EP 17194715
(71) Applicant: PARI GmbH Spezialisten für effektive Inhalation, 82319 Starnberg (DE)
(72) Inventor: Gramann, Jens, 82166 Gräfelfing (DE); Winzen, Andrea, 82166 Gräfelfing (DE); Schuschnig, Uwe, 81371 München (DE)
(74) Representative: Hoffmann Eitle

(57) **Abstract**

Method of generating an aerosol by means of an inhalation device for inhalation by a human or animal body, the method comprising providing liquid in the inhalation device, chemically generating gas bubbles in the liquid, bursting of the gas bubbles at the surface of the liquid, thereby generating an aerosol.

## Description

### Technical field

The present invention relates to a method of generating aerosol in an inhalation device (inhalation therapy device) for inhalation by a human or animal body, and to an inhalation device for generating aerosol and for guiding the aerosol to a human or animal body for inhalation.

It is generally known to nebulize therapeutically effective or active agent-containing liquids into an aerosol consisting of respirable particles by means of an aerosol generator. An aerosol generator is a device that is configured to generate an aerosol. An aerosol generator is preferably a device that is configured to generate liquid droplets and to mix these with a gas. The generated aerosol is offered to a patient for inhalation in the course of an inhalation therapy, as a result of which the therapeutically effective liquid or active agent reaches the respiratory tract of the patient.

Various ways to atomize or nebulize liquid to obtain an aerosol are known. An aerosol generator or inhalation device preferably comprises a nebulizer, an atomizer, a humidifier, a compressed air nebulizer, a jet atomizer, an electronic nebulizer, an ultrasonic nebulizer, an electrohydrodynamic nebulizer, an electrostatic nebulizer, a membrane nebulizer, a nebulizer having a vibrating membrane, an electronic nebulizer having a vibrating membrane, a mesh nebulizer, a nozzle nebulizer, an inhaler (MDI), a powder atomizer (DPI) or a combination thereof. The aerosol generating device might be configured for use with ventilators.

In general, when atomizing liquids, energy has to be applied to the system, so as to generate against the surface tension/cohesion of liquid a larger surface of an instable lamella or a stream, which bursts or collapses afterwards into a number of droplets. The droplets (herein also referred to as particles) form the aerosol for inhalation by the patient.

### Prior art

Such methods for generating aerosol are disclosed in WO 2016/015889 A1 and EP 1 304 131 A1, for example.

However, in the prior art devices, relatively costly parts, which have to be precisely and accurately worked and positioned, are provided, so as to ensure that suitable aerosol generation is possible. Hence, prior art devices involve high accuracy as regards the manufacture and relatively high costs.

### Description of the invention

The object forming the basis of the present invention is to improve the known methods in terms of cost efficiency and manufacture efforts. In particular, a simpler device, including lower requirements as to accuracy and precision of the parts should be provided.

This object is solved by the invention having the features of claim 1. According to claim 1, a method of generating aerosol in an inhalation device for inhalation by a human or animal body is provided, wherein the method comprises providing liquid in the inhalation device, chemically generating gas bubbles in the liquid, bursting of gas bubbles at the surface of the liquid, thereby generating an aerosol. Providing liquid in the inhalation device may, preferably, comprise filling liquid into the inhalation device.

According to claim 10, an inhalation device for generating aerosol and for guiding aerosol to a human or animal body for inhalation is provided. Preferably, the inhalation device is disposable or a single-use inhalation device. The inhalation device comprises an inlet opening for intake of carrier gas, a housing configured to receive liquid and a bubble generating means for generating bubbles in the liquid, wherein the bubble generating means is configured to chemically generate gas bubbles in the liquid, such that gas bubbles burst at the surface of the liquid, thereby generating aerosol (including droplets of the liquid and the intaken carrier gas). An outlet for guiding the carrier gas including the aerosol to the human or animal body for inhalation is also provided.

Hence, the present invention is based upon the idea of forming bubbles in the liquid to be atomized. The aerosol generator or atomizer may basically include a bubble generating means. The bubbles, at the surface of the liquid, form a dome and project from the liquid. The dome is a liquid lamella and is formed on the surface of the liquid. It bursts or collapses and, thereby, generates droplets in the gas, in particular air, above the surface of the liquid. The aerosol comprising the droplets and the carrier gas can be inhaled by the patient. In other words, the aerosol is generated by bursting of the bubbles at the surface of the liquid, without additional or supplement burst supporting means. Preferably, the present invention is free from supplementary or additional burst supporting means, in particular mechanically burst supporting means. The bubbles automatically burst at the surface of the liquid and directly generate the aerosol by releasing the droplets into the carrier gas.

Since the aerosol is formed by means of bubbles that burst on the surface of the liquid, there is no need to provide costly, precisely manufactured and positioned parts for generating the aerosol. For example, no nozzle or membrane is needed for atomizing the liquid.

According to the invention, a simple and robust device can be provided. It is not necessary to provide movable parts, electrical parts and electronics. Further, it allows for silent inhalation. It may be used for a single use and may be a disposable inhalation product.

The surface of the liquid is in particular the upper surface of the liquid.

The generation of aerosol is preferably based on atomizing or nebulizing of liquid.

Aerosols are preferably mixtures of liquid suspended particles (droplets) and a (carrier) gas.

The liquid may comprise a first liquid having a first density and a second liquid having a second density which is lower than the first density, wherein the first and second liquids are (substantially) immiscible (including hardly miscible liquids). Preferably, the second liquid is provided above the first liquid and/or provided at the surface of the liquid comprising the first and second liquids in the inhalation device, at least prior to generating the aerosol.

The aerosol is inhaled by the patient, preferably into the mouth. Aerosols are preferably provided for application on or in parts of the human or animal body such as the skin, body cavities, body orifices, the nose, the paranasal sinuses, the maxillary sinus, the frontal sinus, the sphenoidal sinus, the ethmoidal cells, the throat, the larynx, the trachea, the lungs, the stem bronchus, the bronchi, the bronchioles, the pulmonary alveoli, the joints or the abdominal cavity. Aerosols can be used to diagnose, prevent or treat diseases in humans and animals or to immunize humans or animals against diseases.

The present invention also refers to the use of an inhalation device, and to a method of inhaling aerosol, for example, as detailed at the end of the description.

Preferably, according to the method of generating aerosol, the step of rising of the gas bubbles to the surface of the liquid is comprised. In particular, the bubbles may be generated below the surface of the liquid and rise to an upper surface of the liquid.

In a preferred embodiment, the method comprises the step of bringing a bubble generating means in contact with the liquid, or liquid in contact with the bubble generating means. For generating bubbles in the liquid, it is either the liquid that can be brought in contact with the bubble generating means, or, alternatively, the liquid can be brought into contact with the bubble generating means. In particular, the liquid or the bubble generating means does not have to be brought into contact with each other at one moment. It is conceivable that the contact between the bubble generating means and the liquid is established step by step. In particular, liquid can be brought in contact with the bubble generating means little by little, i.e. gradually, so as to define the amount of liquid in which the bubbles are generated. It is also conceivable that the bubble generating means is little by little, gradually, brought into contact with the liquid. This provides the advantage to dose the amount of liquid to be atomized and the amount of bubbles to be generated.

Preferably, the bubble generating means is immersed in the liquid, further preferably, entirely immersed in the liquid.

The liquid and/or the bubble generating means may comprise an active agent. In particular, it is conceivable that the active agent may be selected from the group consisting of a salt solution; anti-inflammatory agents; anti-infective agents; antiseptics; prostaglandins; endothelin receptor agonists; phosphodiesterase inhibitors; beta-2-sympathicomimetics; decongestants; vasoconstrictors; anticholinergics; immunoglobulins (e.g. Ig, IgG, IgA, IgM); immunomodulators; mucolytics; anti-allergic agents; antihistaminics; mast-cell stabilizing agents; tumor growth inhibitory agents; wound healing agents; local anaesthetics; antioxidants; oligonucleotides; peptides; proteins; vaccines; vitamins; plant extracts; cholinesterase inhibitors; vasoactive intestinal peptide; serotonin receptor antagonists; heparins; glucocorticoids; leucotriene antagonists; antibiotics; antifungals; antivirals; cytostatics; oligonucleotides; xanthin derived agents; natural products or any combination thereof. The active agent may be used in the form of a suspension, a solution, a colloidal formulation (i.e., liposomal), etc.

An active agent in the sense of the invention is therefore also an ingredient which is not an active pharmaceutical ingredient as such, but another ingredient with beneficial effect regarding health, well-being or quality of life of the user. Such another ingredient may be selected from the group consisting of a salt, salt solution, surface active ingredients, osmotic active ingredients, essential oils, plant extracts or ingredients or any combination thereof.

Examples of potentially useful anti-inflammatory agents are glucocorticoids and non-steroidal anti-inflammatory agents, such as betamethasone, beclomethasone, budesonide, ciclesonide, dexamethasone, desoxymethasone, fluoconolone, acetonide, fluocinonide, flunisolide, fluticasone, icomethasone, rofleponide, triamcinolone acetonide, fluocortin butyl, hydrocortisone, hydroxycortisone-17-butyrate, prednicarbate, 6-methylprednisolone aceponate, mometasone furoate, dehydroepiandrosterone-sulfate (DHEAS), elastane, prostaglandin, leukotriene, bradykinin antagonists, non-steroidal anti-inflammatory drugs (NSAIDs), such as ibuprofen including any pharmaceutically acceptable salts, esters, isomers/stereoisomers, diastereomers, epimers, solvates or other hydrates, prodrugs, derivatives, or any other chemical or physical forms of active agents comprising the respective active moieties.

Examples of anti-infective agents, whose class or therapeutic category is herein understood as comprising compounds which are effective against bacterial, fungal, and viral infections, i.e. encompassing the classes of antimicrobials, antibiotics, antifungals, antiseptics, and antivirals, are penicillins, including, but not limited to, benzylpenicillins (penicillin-G sodium, clemizone penicillin, benzathine penicillin G), phenoxypenicillins (penicillin V, propicillin), aminobenzylpenicillins (ampicillin, amoxycillin, bacampicillin), acylaminopenicillins (azlocillin, mezlocillin, piperacillin, apalcillin), carboxypenicillins (carbenicillin, ticarcillin, temocillin), isoxazolyl penicillins (oxacillin, cloxacillin, dicloxacillin, flucloxacillin), and amiidine penicillins (mecillinam); cephalosporins, including, but not limited to, cefazolins (cefazolin, cefazedone); cefuroximes (cefuroxim, cefamandole, cefotiam), cefoxitins (cefoxitin, cefotetan, latamoxef, flomoxef), cefotaximes (cefotaxime, ceftriaxone, ceftizoxime, cefmenoxime), ceftazidimes (ceftazidime, cefpirome, cefepime), cefalexins (cefalexin, cefaclor, cefadroxil, cefradine, loracarbef, cefprozil), and cefiximes (cefixime, cefpodoxim proxetile, cefuroxime axetil, cefetamet pivoxil, cefotiam hexetil), loracarbef, cefepim, clavulanic acid/amoxicillin, ceftobiprole; synergists, including, but not limited to, beta-lactamase inhibitors, such as clavulanic acid, sulbactam, and tazobactam; carbapenems, including, but not limited to, imipenem, cilastin, meropenem, doripenem, tebipenem, ertapenem, ritipenam, and biapenem; monobactams, including aztreonam; aminoglycosides, such as apramycin, gentamicin, amikacin, isepamicin, arbekacin, tobramycin, netilmicin, spectinomycin, streptomycin, capreomycin, neomycin, paromoycin, and kanamycin; macrolides, including erythromycin, clarythromycin, roxithromycin, azithromycin, dithromycin, josamycin, spiramycin and telithromycin; gyrase inhibitors or fluroquinolones, including, but not limited to, ciprofloxacin, gatifloxacin, norfloxacin, ofloxacin, levofloxacin, perfloxacin, lomefloxacin, fleroxacin, garenoxacin, clinafloxacin, sitafloxacin, prulifloxacin, olamufloxacin, caderofloxacin, gemifloxacin, balofloxacin, trovaf loxacin, and moxifloxacin; tetracyclins, including tetracyclin, oxytetracyclin, rolitetracyclin, minocyclin, doxycycline, tigecycline and aminocycline; glycopeptides, including, but not limited to, vancomycin, teicoplanin, ristocetin, avoparcin, oritavancin, rarnoplanin, and peptide; polypeptides, including plectasin, dalbavancin, daptomycin, oritavancin, ramoplanin, dalbavancin, telavancin, bacitracin, tyrothricin, neornycin, kanamycin, rnupirocin, paromomycin, polymyxin B and colistin; sulfonamides, including, but not limited to, sulfadiazine, sulfamethoxazole, sulfalene, co-trimoxazole, co-trimetrol, co-trimoxazine, and co-tetraxazine; azoles, including clotrimazole, oxiconazole, miconazole, ketoconazole, itraconazole, fluconazole, metronidazole, tinidazole, bifonazol, ravuconazol, posaconazol, voriconazole, and ornidazole and other antifungals including, but not limited to, flucytosin, griseofulvin, tolnaftal, naftifin, terbinafin, amorolfin, ciclopiroxolamin, echinocandins, such as micafungin, caspofungin, anidulafungin; nitrofurans, including, but not limited to, nitrofurantoin and nitrofuranzone; polyenes, including, but not limited to, amphotericin B, natamycin, nystatin, flucytosine; other antibiotics, including, but not limited to, tithromycin, lincomycin, clindamycin, oxazolindiones (linzezolids), ranbezolid, streptogramine A+B, pristinamycin A+B, dalfopristin/quinupristin (Synercid), Virginiamycin A+B, chloramphenicol, ethambutol, pyrazinamid, terizidon, dapson, prothionamid, fosfomycin, fucidinic acid, rifampicin, isoniazid, cycloserine, terizidone, ansamycin, lysostaphin, iclaprim, mirocin B17, clerocidin, filgrastim, formycin, and pentamidine; antivirals, including, but not limited to, aciclovir, ganciclovir, birivudin, valaciclovir, zidovudine, didanosin, thiacytidin, stavudin, lamivudin, zalcitabin, ribavirin, nevirapirin, delaviridin, trifluridin, ritonavir, saquinavir, indinavir, foscarnet, amantadin, podophyllotoxin, vidarabine, tromantadine, and proteinase inhibitors, siRNA based drugs; antiseptics, including, but not limited to, acridine derivatives, iodinepovidone, benzoates, rivanol, chlorhexidine, quarternary ammoniurn compounds, cetrirnides, biphenylol, clorofene, and octenidine; interferones (alpha, beta, gamma), tumor necrosis factors, cytokines, interleukines; immunmodulators including, but not limited to, methotrexat, azathioprine, cyclosporine, tacrolimus, sirolimus, rapamycin, mofetil; mofetil-mycophenolate cytostatics and metastasis inhibitors; alkylants, such as nimustine, melphanlane, carmustine, lomustine, cyclophosphosphamide, ifosfamide, trofosfamide, chlorambucil, busulfane, treosulfane, prednimustine, thiotepa; antimetabolites, e.g. cytarabine, fluorouracil, methotrexate, rnercaptopurine, tioguanine; alkaloids, such as vinblastine, vincristine, vindesine; antibiotics, such as alcarubicine, bleomycine, dactinomycine, daunorubicine, doxorubicine, epirubicine, idarubicine, mitomycine, plicamycine; complexes of transition group elements (e.g. Ti, Zr, V, Nb, Ta, Mo, W, Pt) such as carboplatinum, cis-platinum and metallocene compounds such as titanocendichloride; amsacrine, dacarbazine, estramustine, etoposide, beraprost, hydroxycarbamide, mitoxanthrone, procarbazine, temiposide; paclitaxel, gefitinib, vandetanib, erlotinib, poly-ADPribose-polymerase (PRAP) enzyme inhibitors, banoxantrone, gemcitabine, pemetrexed, bevacizumab, ranibizumab.

Examples of potentially useful mucolytics are DNase, P2Y2-agonists (denufosol), drugs affecting chloride and sodium permeation, such as N-(3,5-Diamino-6-chloropyrazine-2-carbony)-N'-{4-[4-(2,3-dihydroxypropoxy)phenyl]butyl}guanidine methanesulfonate (PARION 552-02), heparinoids, guaifenesin, acetylcysteine, carbocysteine, ambroxol, bromhexine, tyloxapol, lecithins, myrtol, surfactant, and recombinant surfactant proteins.

Examples of potentially useful vasoconstrictors and decongestants which may be useful to reduce the swelling of the mucosa are phenylephrine, naphazoline, tramazoline, tetryzoline, oxymetazoline, fenoxazoline, xylometazoline, epinephrine, isoprenaline, hexoprenaline, and ephedrine.

Examples of potentially useful local anaesthetic agents include, but not limited to, benzocaine, tetracaine, procaine, lidocaine and bupivacaine.

Examples of potentially useful antiallergic agents include, but not limited to, the afore-mentioned glucocorticoids, cromolyn sodium, nedocromil, cetrizin, loratidin, montelukast, roflumilast, ziluton, omalizumab, heparinoids and other antihistamins, including, but not limited to, azelastine, cetirizin, desloratadin, ebastin, fexofenadin, levocetirizin, loratadin.

Examples of potentially useful anticholinergic agents include, but not limited to, ipratropium bromide, thiotropium bromide, oxitropium bromide, glycopyrrolate.

Examples of potentially useful beta-2-sympathicomimetic agents include, but not limited to, salbutamol, fenoterol, formoterol, indacaterol, isoproterenol, metaproterenol, salmeterol, terbutaline, clenbuterol, isoetarine, pirbuterol, procaterol, ritodrine.

Examples of xanthine derived agents include, but not limited to, theophylline, theobromine, caffeine.

Antisense oligonucleotides are short synthetic strands of DNA (or analogs) that are complimentary or antisense to a target sequence (DNA, RNA) designed to halt a biological event, such as transcription, translation or splicing. The resulting inhibition of gene expression makes oligonucleotides dependent on their composition useful for the treatment of many diseases and various compounds are currently clinically evaluated, such as ALN-RSVOl to treat the respiratory syncytical virus by, AVE-7279 to treat asthma and allergies, TPI-ASM8 to treat allergic asthma, 1018-ISS to treat cancer.

Examples of potentially useful peptides and proteins include, but not limited to, antibodies against toxins produced by microorganisms, antimicrobial peptides such as cecropins, defensins, thionins, and cathelicidins.

In more preferred embodiments, the active agent may be selected from the group consisting of a salt such as NaCl, anorganic and organic zinc salts/compounds, salts of bismuth and selen, or a salt solution thereof; essential oils; plant extracts or ingredients (also referred to as natural product) such as Ectoine (methyl-1,4,5,6-tetrahydropyrimidine-4-carboxylic acid), plant extracts from chamomile, hamamelis, echinacea, calendula, thymian, papain, pelargonium, pine trees, myrtol, pinen, limonen, cineole, thymol, mental, camphor, tannin, alpha-hederin, bisabolol, lycopodin, vitapherole; mucolytics as defined above; or any combination thereof.

The active agent may comprise a drug that cannot be administered orally. The active agent may be for topical or systemic delivery via either a nasal or a pulmonary route.

Most preferably, the active agent may comprise or consist of a salt solution (saline solution) or essential oil(s). The saline solution may be more preferably saturated with NaCl. For instance, a solution having a NaCl concentration of 36% by weight is saturated at room temperature. Alternatively, the saline solution may have a salinity of less than or equal to 40% by weight or 35% by weight, preferably a salinity between 0.5 to 20% by weight, more preferably a salinity between 0.9 to 7% by weight.

The active agent may also be a suspension or an emulsion.

Preferably, the liquid is water or comprises water (aqueous solution). However, other liquids are also conceivable.

The gas that is used for forming the bubbles can be air, preferably pressurized air. Other gases and gas mixtures are also possible, for example carbon dioxide or oxygen.

Preferably, the liquid or the gas is heated. For example, generation of gas bubbles may be supported by increasing the temperature of the liquid, and so as to support a chemical reaction as specified below. The inhalation device may comprise heating means to heat liquid. This supports the generation of bubbles.

The gas bubbles can be generated by flowing gas through porous materials, a fabric and/or a filament. Gas is guided through the porous material, before it contacts the liquid. Also metal, plastics or minerals in the form of a sintered, porous entity are conceivable. This kind of bubble generation can be regarded as a mechanical bubble generation. For example, the porous material may be selected of the group consisting of stainless steel, bronze, ceramics, sintered glass, and sintered plastics (such as polyethylene). For example, the fabric may be selected of the group consisting of polyamide, polyester and polypropylene, and/or the filament may be selected of the group consisting of polyamide, polyester and polypropylene. Preferably, the average pore diameter of the porous material is in the range between 1 µm and 0.1 mm. In particular, in case of metal sintered filters, the pore diameter may be about 1 µm.

Alternatively or additionally, the bubbles can be generated on the basis of a chemical reaction generating an inert gas such as carbon dioxide, oxygen or nitrogen. For example, carbon dioxide may be released when subjecting a salt of carbonic acid (such as sodium (hydrogen)carbonate, and/or potassium (hydrogen)carbonate) a reaction with an acid in the presence of water. For instance, the salt of carbonic acid may include sodium carbonate and potassium carbonate; sodium carbonate and/or potassium hydrogen carbonate; sodium hydrogen carbonate and/or potassium carbonate; sodium carbonate and/or sodium hydrogen carbonate; potassium carbonate and/or potassium hydrogen carbonate. Preferred mixtures are 20/80, 40/60, 50/50, 60/40, 80/20% by weight.

In a preferred embodiment, the salt of carbonic acid may comprise or consist (essentially) of an alkali carbonate such as sodium carbonate and/or potassium carbonate. Preferably, no alkali hydrogen carbonate is used as additional salt of carbonic acid in this embodiment. The use of an alkali carbonate such as sodium carbonate and/or potassium carbonate improves the taste and smell of the aerosol by increasing the pH value thereof, i.e. a less acidic aerosol is obtained. A less acidic aerosol is better tolerated and does not cause any irritation of the throat or respiratory tract.

In a particularly preferred embodiment, the salt of carbonic acid may comprise or consist (essentially) of sodium carbonate and/or potassium carbonate. Both potassium carbonate and sodium carbonate are highly water soluble.

Without being bound by this theory, the effect of an improved taste mentioned above may be explained as follows: Conventional effervescent formulations consist largely of sodium hydrogen carbonate (NaHCO₃) in a stochiometric ratio to a suitable organic acid (e.g. citric acid, tartaric acid or malic acid). This has the advantage that more carbon dioxide is produced as compared to when using the corresponding carbonate salt (Na₂CO₃). However, the solubility of citric acid is higher than that of hydrogen carbonate salts. Moreover, hydrogen carbonate salts decompose at higher temperatures (>50°C) to CO₂ and the corresponding carbonate salt. Accordingly, a fresh effervescent solution is acidic at the beginning and neutralized only towards the end of the reaction. An alkali carbonate salt shows a considerably higher solubility than the corresponding alkali hydrogen carbonate and does not release CO₂ at elevated temperatures It therefore follows that an alkali carbonate is able to neutralize the excess of dissolved citric acid at the beginning of the reaction to a greater extent than an alkali hydrogen carbonate, thereby resulting in a less acidic solution and a less acidic aerosol. Additionally, the less acidic aerosol may be caused by the decomposition of alkali hydrogen carbonates to carbonates under release of carbon dioxide at temperatures above 50°C. Due to this decomposition, an aerosol may be formed at the beginning of the reaction, but citric acid is however not neutralized. The acid may be preferably a solid acid such as citric acid, tartaric acid or malic acid. Most preferably, the acid is citric acid. In one embodiment, the solid acid is granulated and optionally coated with a coating agent for delaying the solution of the solid acid. For instance, the coating agent may be a food grade shellac (also referred to as confectioners glaze).

The bubbling generating means and liquid are brought in contact, wherein gas is generated by means of reaction of a substance with the liquid or through thermal dissolution or decomposition. The bubbling generating means can, for example, be in the form of powder, a pill, pellet and/or compressed powder. Preferably, the bubbling generating means can be an effervescent tablet. More preferably, the bubbling generating means can be an effervescent tablet comprising a salt of carbonic acid (such as sodium (hydrogen)carbonate, and/or potassium (hydrogen)carbonate, see above), an acid (such as citric acid, tartaric acid or malic acid) and optionally an active agent as defined above. An effervescent tablet (also referred to as carbon tablet) is a tablet which is designed to dissolve in water, and release carbon dioxide. Effervescent tablets are commonly known in the art. For example, powder can be comprised in porous wraps that carry the powder. This kind of bubble generation can be regarded as a chemical bubble generation.

In a particularly preferred embodiment, the bubble generating means is an effervescent tablet comprising or consisting (essentially) of sodium and/or potassium carbonate and citric acid (or another suitable acid). Preferred mixtures of sodium and/or potassium carbonate to citric acid (or another suitable acid) are 20/80, 40/60, 50/50, 60/40, or 80/20% by weight. A most preferred mixture is 50/50% by weight.

The bubble generating means may comprise agent (s), such as salt(s), active agent(s), flavor or odorous substance(s), dye(s), binding agent(s), defoaming substance(s), or substances that accelerate or delay solution of the substance. A suitable defoaming substance is silicone oil, for example.

In a preferred embodiment, the bubble generating means comprises additives for increasing the aerosol production by reducing foam formation and/or formation of large droplets. For instance, defoaming agents such as emulsified oil, silicone oil, lecithin, magnesium stearate and the like may be used.

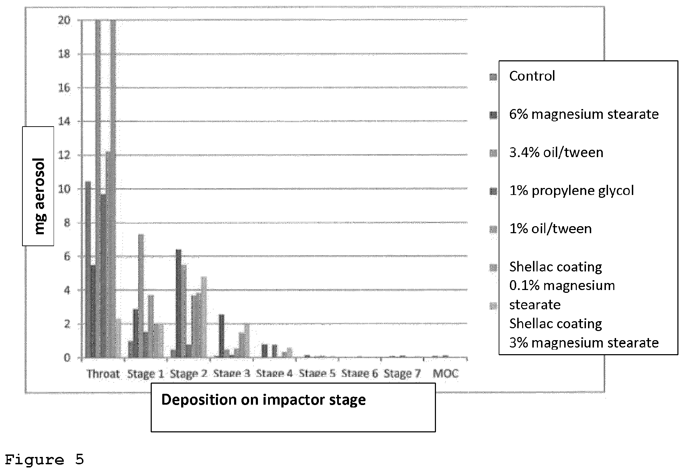

Defoaming agents such as emulsified oil, silicone oil, lecithin, magnesium stearate and the like may also affect the size of the formed droplets as illustrated in Figure 5. Figure 5 shows the release kinetics of aerosols formed from several effervescent tablets in accordance with the present invention in an impactor experiment. Specifically, a next generation cascade impactor method as described in the USP<601> and <1601> method was used for analyzing the amount of aerosols formed from said effervescent tablets as a function of their deposition on the impactor stages. In particular, said impactor was operated at 15 1/min flow rate and the induction port was connected to a vessel containing water mixed with dye. The effervescent tablet to be analyzed was added to the water and the resulting aerosol was sucked into the impactor for about 30 seconds. The amount of dye deposited on the impactor stages was then determined by UV-VIS photometry and the amount of aerosol calculated from these values.

The depositions on the impactor stages are indicative for the size of aerosol droplets: the smaller the droplet size, the higher deposition stages may be reached. The effervescent tablets are formed from sodium carbonate/citric acid/mannitol in a ratio of 10.4/12.5/7.5 gram (mass ratio) as a basis and supplemented with the indicated additive (s). The pH of all tested effervescent tablets was higher than the pH of common effervescent tables comprising a high amount of sodium hydrogen carbonate and was therefore less acidic and less irritating for the respiratory tract upon inhalation.

Preferably, the inhalation device comprises the bubble generating means, wherein the housing accommodates the bubble generating means. The provision of the bubble generating means inside the inhalation device is advantageous. The bubble generating means, however, may also be provided outside the inhalation device and inserted in to the inhalation device prior to the use of the inhalation device only.

The inhalation device may comprise a guide for guiding the carrier gas at least partially along the surface of the liquid. This provides the advantage that the aerosol that is generated on the surface of the liquid can be taken with the carrier gas, so as to enrich the carrier gas that is inhaled by the patient. The carrier gas carries the droplets to the outlet. Hence, the effects of the inhalation can be improved.

In particular, the guide may comprise a cap, wherein the cap extends close to the surface of the liquid. The provision of such cap allows for guiding the carrier gas close to the surface of the liquid.

Preferably, the carrier gas is ambient air.

In a preferred embodiment, a terminal for connection with a gas source for introducing gas into the liquid, i.e. the gas generating means, is provided. By means of the gas source, a mechanical option for generating bubbles is realized. Preferably, pressurized gas is used for introducing gas into the gas generating means.

Preferably, an insulating member is provided, wherein the insulating member at least partially surrounds the housing. The insulating member is in particular advantageous in case the liquid is heated or if heat is produced. The insulating material can, for example, be neoprene. In particular, the insulating member can be configured for keeping the fluid and/or the gas at a desired temperature, preferably for keeping the fluid and/or gas at a temperature above room temperature, more preferably above 25°C or 30°C or 35°C. In a preferred embodiment, the insulating member surrounds the housing in its entirety.

### Brief description of the drawings

Figure 1 shows a preferred embodiment of the present invention;
Figure 2 shows another preferred embodiment of the present invention;
Figure 3 schematically shows the physical background underlying the present invention.
Figure 4 shows an aerosol spectrum.
Figure 5 shows the release kinetics of aerosols formed in accordance with certain embodiments of the present invention.

### Detailed description of preferred embodiments of the invention

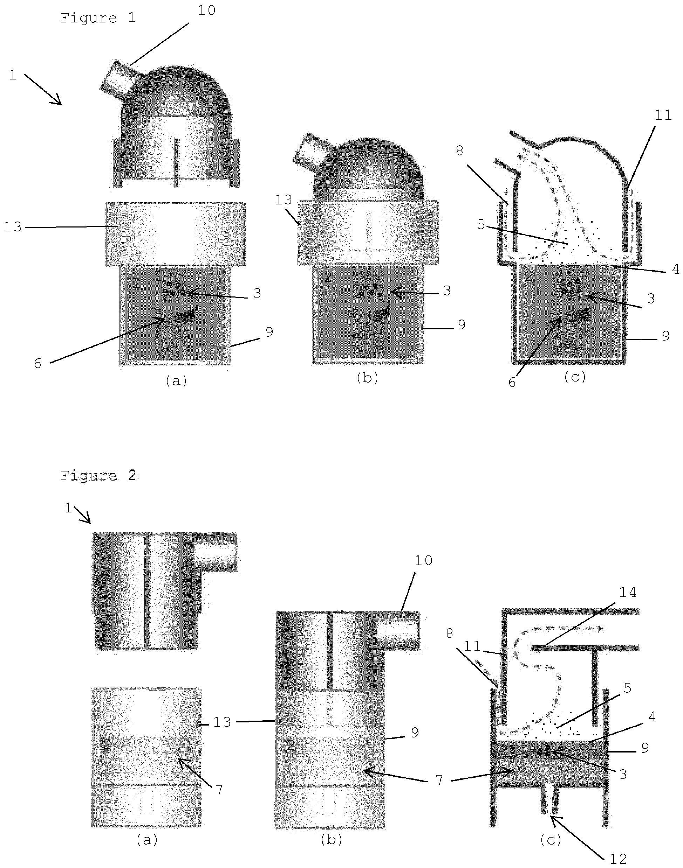

In each of Figures 1 and 2, Figure (a) shows a view of separated parts of the inhalation device 1, Figure (b) shows the complete and assembled inhalation device 1, and Figure (c) shows a cross-section of the inhalation device 1 including arrows indicating the air flow upon breathing and sucking in.

Figure 1 depicts the inhalation device 1 comprising an outlet 10 for guiding the air to the patient's mouth. The liquid 2, for example, may comprise medication. The housing 9 accomodates the liquid 2 and a bubble generating means 6. In this embodiment, the bubbles 3 are generated by means of a chemical reaction. Either the pellet 6 as bubble generating means is brought into the housing 9 comprising the liquid 2 or the liquid 2 is poured into the housing 9 in which the pellet 6 is provided.

Hence, the bubble generating means 6 of the first embodiment is configured to chemically generate the gas bubbles 3. The bubble generating means may, for example, be a pill or pellet 6. The pill or pellet 6 may, for example, comprise a salt of carbonic acid (such as sodium (hydrogen)carbonate, and/or potassium (hydrogen)carbonate), an acid and optionally an active agent. When coming into contact with the liquid 2, which may be preferably water or an aqueous solution, carbon dioxide is formed as inert gas which may be able to generate gas bubbles in the liquid 2.

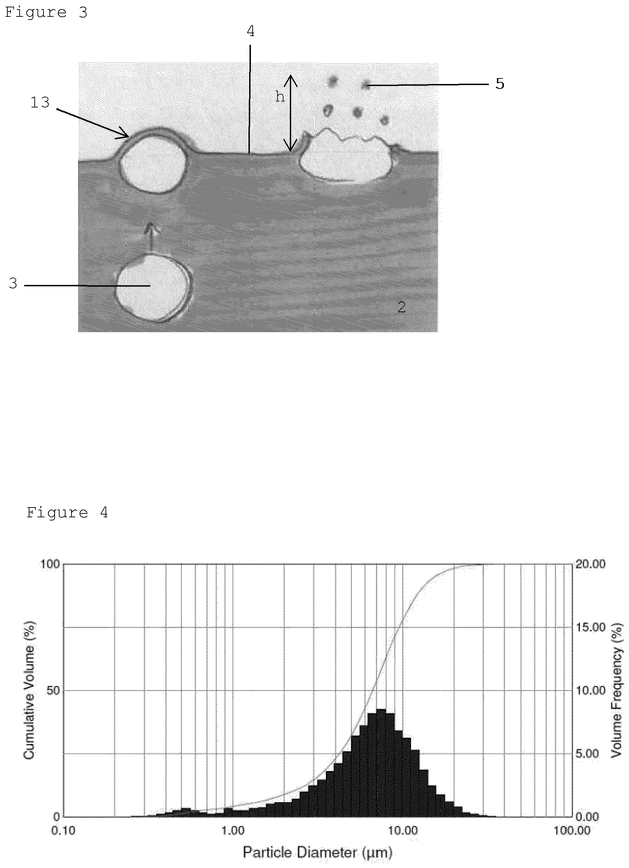

Liquid 2 has been filled into the inhalation device 1, in particular in the housing 9, which forms a chamber or reservoir. By means of a chemical reaction between the bubble generating means 6 and the surrounding liquid 2, gas bubbles 3 are formed. The gas bubbles 3 (automatically) rise to the surface 4 of the liquid and are believed to form domes that partially project from the surface 4 of the liquid. This will be explained in detail in connection with Figure 3.

The gas bubbles burst at the surface 4 of the liquid 2 and generate droplets 5, and, thereby, aerosol. An inlet opening 8, as indicated in Figure 1(c), is provided, so that carrier gas (ambient air) can enter the inhalation device 1. When the patient sucks air upon breathing, ambient air is taken in through the inlet opening 8.

A cap 11 is formed as a guide for guiding the ambient air along the surface 4 of the liquid 2. To avoid that, due to gravity, a number of the droplets 5 fall back in the liquid 2, the stream of carrier gas (air stream) is used to collect the droplets 5 at the surface 4 of the liquid 2. The cap 11 extends at least partially close to the surface 4 of the liquid and, thus, guides the ambient air at least partially along the surface 4 of the liquid. In this embodiment, the cap 11 and the housing 9 partially overlap and form, in the overlap, the inlet opening 8. In this embodiment, the inlet opening 8 is formed in a circle, so that ambient air is guided from all angles onto the surface of the liquid 4 inside the housing 9.

As indicated in Figure 1(c), ambient air enters the inhalation device 1 through the inlet opening 8 and enriches with droplets 5 generated by bursting of the bubbles 3 on the surface. Preferably, heating means (not shown) are provided.

In Figures 1(a) and 1(b), the insulating member 13 which partially surrounds the housing is shown. Such member 13 can in particular be used if the liquid 2 is heated or heat is generated during the chemical reaction.

In the embodiment of Figure 2, the bubbles are generated on a mechanical basis. More specifically, the bubble generating means 7 comprises porous material, which is positioned below the liquid 2. The housing 9 accommodates the bubble generating means 7 and further comprises a terminal 12 for connection with a gas source for introducing gas into the bubble generating means 7. When gas advances through the porous material 7, which forms the bubble generating means, bubbles are generated in the liquid 2 and rise to the surface 4 of the liquid 2. At the surface 4, the bubbles 3 burst, whereby they generate aerosol including droplets 5 of the liquid 2.

Also in the embodiment of Figure 2, an outlet 10 for guiding the aerosol to the patient's mouth is provided. An insulating member 13 may also be provided.

In Figure 2(c), the flow of ambient air into the inhalation device 1 is indicated by the arrows. Ambient air enters through the inlet opening 8 onto the surface 4 of the liquid, where it enriches with droplets 5.

In the embodiment of Figure 2, a deflection plate 14 for filtering large droplets 5 out of the aerosol that is inhaled is provided.

Figure 3 schematically shows the liquid 2 in which three different stages or stadia of bubbles 3 are shown. The lower bubble 3 on the left side rises in the liquid. The upper, left bubble 3 has reached the upper surface 4 and forms a dome-shaped bubble. It has a thin lamella or film 13 of the liquid. The dome-shaped lamella 13 projects from the liquid surface 4. The right bubble 3 is in the progress of bursting, as indicated by the droplets 5 discharged into the air. The liquid lamella 13 of the film surrounding the bubble 3 collapses and is released in the form of the small droplets 5.

When a bubble 3 bursts, film droplets and jet droplets can be formed. Without wishing to be bound to any theory, depending on the density of the liquid, film and jet droplets can be formed.

It was observed that jet droplet are in particular formed of a first liquid having a relatively high density (such as higher that the second density of a second liquid) and/or being provided below the second liquid. For jet droplets to be formed, liquid flows to the middle of the bubble and forms droplets. It was also observed that film droplets are in particular formed of a second liquid having a relatively low density (such as lower that the first density of a first liquid) and/or being provided above the first liquid. Film droplets are formed when the film ruptures. It is conceivable to filter off the larger jet droplets from the aerosol (by means known from the prior art), for example, if only smaller film droplets are desired for inhalation.

The diameter of the bubbles 3 is larger than 1 mm. Typically, the diameter of a droplet 5 (or the average diameter of the droplets) is between 1 µm and 100 µm, preferably in the range between 1 and 10 µm, preferably between 1 and 5 µm. According to experiments, the height h, as indicated in Figure 3, up to which the droplets 5 are released or shoot out, is in the range of 1 or a few cm.

The diameter of the bubbles may be below 0.1 mm, wherein the diameter of the jet droplets would be about 10 to 15 % of the diameter of the bubble. The height h would, in this case, be about 100 times the diameter of the bubble.

Usually, jet droplets are larger than film droplets. For example, a jet droplet can have a diameter of about 50 to 100 µm, wherein a film droplet can have a diameter of about 1 to 50 µm.

In general, it is advantageous that the diameter of a bubble 3 is larger than 0.5 mm, to support that a sufficient amount of droplets is generated.

The liquid may comprise the first liquid having the first density and the second liquid having the second density which is lower than the first density, wherein the first and second liquids are (substantially) immiscible (including hardly miscible liquids). Preferably, the second liquid is provided above the first liquid and/or provided at the surface of the liquid comprising the first and second liquids in the inhalation device, at least prior to generating the aerosol. Preferably, the first liquid comprises water, and the second liquid comprises an active agent. It is also conceivable that the first and second liquids comprise different active agents.

The amount of the second liquid is preferably such that the bubbles/each bubble at least partially extend(s) into the first and second liquids upon burst of the bubble. Hence, the droplets are at least partially surrounded by the first and second liquids.

If the bubble generating means is provided within the first liquid having the first density, and the second liquid is provided at least partially above the second liquid, a bubble can be embedded in or can be surrounded by the first and second liquids. Without wishing to be bound to theory, first droplets are formed on the basis of the first liquid and second droplets are formed on the basis of the second liquid. If the first liquid has a higher density that the second liquid, the first droplets are (on average) larger than the second droplets (on average). Put differently, the first liquid provides for larger (first) droplets than the second liquid leading to smaller (second) droplets.

A number of applications are conceivable: For example, the droplet size has an effect on the application distance, i.e. on how far a droplet passes through the human body: Larger droplets will only reach the upper respiratory tract (nose), wherein smaller droplets reach the lower respiratory tract (lung). Hence, depending on which part of the respiratory tract shall primarily be treated with medicament, the first and second liquids can be selected accordingly to provide for appropriate first and second droplets. If only the second (smaller) droplets are desired for inhalation, the first droplets can be removed from the aerosol prior to inhalation, by means of a filter or a buffle, for example.

A preferred application could also be for damageable medicaments and/or active agents, in particular in the second liquid. If a damageable or sensitive medicament, which might be damaged by application of high shear force, for example, is used as second liquid above the first liquid, it is protected by the first liquid to some extent. This would also be advantageous in case a sensitive (second) liquid is heated by the below first liquid. In this case, the second liquid is not directly heated, but by means of heat conducted by the first liquid.

It may depend on the surface tension and the density of the liquid how and how many droplets are formed. In general, the surface tension and the density of the liquid influence the percentage and the quality of the droplets (film droplets and jet droplets). In particular, according to the present invention, tenside might be disadvantageous and should not be comprised in the liquid. For example, the invention has been put into practice by means of a fizzy tablet as shown in the embodiment of Figure 1 above. The skilled person is able to provide appropriate liquid 2 and bubble generating means 6, 7 so as to obtain bubbles 3 that burst at the surface 4 of the liquid.

Foam may be generated on the surface of the liquid 4. However, foam is not desired, let alone needed for the invention. Preferably, the liquid is basically free from foam generating agents. Hence, generation of aerosol does preferably not involve generation of foam, i.e. is without generation of foam.

Figure 4 shows measurement results for an aerosol spectrum. The measurements show that the average diameter of the droplets was about 6 to 7 µm.

Figure 5 shows the release kinetics of aerosols formed from several effervescent tablets in accordance with the present invention in an impactor experiment (see also explanations above).

The following aspects also relate to the present invention:
1. Use of an inhalation device (1), preferably a disposable or single-use inhalation device, for generating aerosol and for guiding aerosol to a human or animal body for inhalation, the inhalation device comprising:
   a housing (9) configured to receive liquid (2) and a bubble generating means (6, 7) for generating bubbles (3) in the liquid,
   the use comprising:
      Providing liquid (2) in the inhalation device (1),
      Mechanically and/or chemically generating gas bubbles (3) in the liquid (2),
      Bursting of said gas bubbles (3) at the surface (4) of the liquid, thereby generating aerosol.
2. The use of item 1, the inhalation device comprising the bubble generating means, the housing (9) accommodating the bubble generating means (6, 7).
3. The use of item 1 or 2, wherein the bubble generating means (6) includes a powder, a pill and/or a compressed powder.
4. The use of any of the preceding items 1 to 3, wherein the bubble generating means (7) comprises porous material and/or fabric and/or filament configured for gas to be flowed through.
5. The use of any of the preceding items, the inhalation device further comprising heating means to heat liquid.
6. The use of any of the preceding items, the inhalation device further comprising a guide (11) for guiding carrier gas, in particular ambient air, at least partially along the surface (4) of the liquid.
7. The use of item 6, the guide comprising a cap (11), the cap extending close to the surface of the liquid.
8. The use of any of the preceding items, further comprising a terminal (12) for connection with a gas source for introducing gas into the liquid.
9. The use of any of the preceding items, further comprising an insulating member (13) at least partially surrounding the housing (9).
10. The use of any of the preceding items, further comprising rising of the gas bubbles to the surface (4) of the liquid.
11. The use of any of the preceding items, wherein the a bubble generating means (6, 7) is brought in contact with the liquid (2), or liquid in contact with the bubble generating means (6, 7).
12. The use of any of the preceding items, wherein the liquid and/or the bubble generating means (6, 7) comprises an active agent, which preferably comprises or consists of a salt or a salt solution
13. The use of any of the preceding items, comprising heating of liquid and/or of gas before generating the gas bubbles.
14. The use of any of the preceding items, comprising generating of gas bubbles by flowing gas through porous material, fabric and/or filament (7).
15. The use of any of the preceding items, comprising chemical generating of gas bubbles by bringing the bubble generating means (6), preferably in the form of a powder, a pill, and/or compressed powder, in contact with liquid (2).
16. The use of item 15, wherein the active agent is an active agent for the local or systemic treatment of a respiratory disease.

Additionally, the following aspects relate to the present invention:
1. Method of inhaling aerosol by means of an inhalation device (1) by a human or animal body, the method comprising:
   Providing liquid (2) in the inhalation device (1),
   Mechanically and/or chemically generating gas bubbles (3) in the liquid (2),
   Bursting of gas bubbles (3) at the surface (4) of the liquid, thereby generating aerosol including droplets (5) of the liquid, and
   Inhaling the aerosol by the human or animal body.
2. Method of item 1, further comprising the step of rising of the gas bubbles to the surface (4) of the liquid.
3. Method of item 1 or 2, further comprising the step of bringing a bubble generating means (6, 7) in contact with the liquid (2), or liquid in contact with the bubble generating means (6, 7).
4. Method of any of the preceding items, wherein the liquid and/or the bubble generating means (6, 7) comprises an active agent, which preferably comprises or consists of a salt or a salt solution.
5. Method of any of the preceding items, comprising heating of liquid and/or of gas before generating the gas bubbles.
6. Method of any of the preceding items, comprising mechanical generating of gas bubbles by flowing gas through porous material, fabric and/or filament (7).
7. Method of any of the preceding items, comprising chemical generating of gas bubbles by bringing the bubble generating means (6), preferably in the form of a powder, a pill, and/or compressed powder, in contact with liquid (2).
8. Method of item 7, wherein the active agent is an active agent for the local or systemic treatment of a respiratory disease.

Additionally, the following aspects (also in connection with the dependent items 2 to 8) relates to the present invention:
Method of generating aerosol in an inhalation device (1) for inhalation by a human or animal body, the method comprising:
Providing liquid (2) in the inhalation device (1),
Mechanically and/or chemically generating gas bubbles (3) in the liquid (2),
Bursting of gas bubbles (3) at the surface (4) of the liquid, thereby generating aerosol droplets (5) of the liquid.

Additionally, the following aspect (also in connection with the dependent items 10 to 17) relates to the present invention:
Inhalation system comprising inhalation device (1), preferably a disposable or single-use inhalation device, and a bubble generating means, for generating aerosol and for guiding aerosol to a human or animal body for inhalation, the inhalation device comprising
an inlet opening (8) for intake of carrier gas, in particular ambient air,
a housing (9) configured to receive liquid (2) and the bubble generating means (6, 7) for generating bubbles (3) in the liquid,
the bubble generating means (6, 7) being configured to mechanically and/or chemically generate gas bubbles (3) in the liquid (2), such that gas bubbles (3) partially project from the surface (4) of the liquid and burst at the surface of the liquid, and to thereby generate aerosol droplets (5) of the liquid entrained by the carrier gas, and
an outlet (10) for guiding the carrier gas including the aerosol to the human or animal body for inhalation.

### The following numbered items also belong to the invention:

1. Method of generating aerosol in an inhalation device (1), the method comprising:
   Providing liquid (2) in the inhalation device (1),
   Mechanically and/or chemically generating gas bubbles (3) in the liquid (2),
   Bursting of said gas bubbles (3) at the surface (4) of the liquid, thereby generating an aerosol.
2. Method of 1, further comprising the step of rising of the gas bubbles to the surface (4) of the liquid.
3. Method of 1 or 2, further comprising the step of bringing a bubble generating means (6, 7) in contact with the liquid (2), or liquid in contact with the bubble generating means (6, 7).
4. Method of any of the preceding items, wherein the liquid and/or the bubble generating means (6, 7) comprises an active agent, which preferably comprises or consists of a salt or a salt solution.
5. Method of any of the preceding items, comprising mechanical generating of gas bubbles by flowing gas through a porous material, fabric and/or filament (7).
6. Method of any of the preceding items, comprising heating of said liquid and/or said gas before generating the gas bubbles.
7. Method of any of the preceding items, comprising chemical generating of gas bubbles by bringing the bubble generating means (6), preferably in the form of a powder, a pill, and/or compressed powder, in contact with liquid (2).
8. Method of any of the preceding items, wherein the bubble generating means (6) includes an effervescent tablet which optionally comprises an active agent.
9. Method of any of the preceding items, wherein the active agent is an active agent for the local or systemic treatment of a respiratory disease.
10. Inhalation device (1), preferably a disposable or single-use inhalation device, for generating aerosol and for guiding aerosol to a human or animal body for inhalation, the inhalation device comprising
   an inlet opening (8) for intake of carrier gas, in particular ambient air,
   a housing (9) configured to receive liquid (2) and a bubble generating means (6, 7) for generating bubbles (3) in the liquid,
   the bubble generating means (6, 7) being configured to mechanically and/or chemically generate gas bubbles (3) in the liquid (2), such that gas bubbles (3) burst at the surface of the liquid, thereby generating an aerosol, and
   an outlet (10) for guiding the aerosol to the human or animal body for inhalation.
11. The inhalation device of 10, the inhalation device comprising the bubble generating means, the housing (9) accommodating the bubble generating means (6, 7), and/or the inhalation device comprising the liquid (2).
12. The inhalation device of 10 or 11, wherein the bubble generating means (6) includes a powder, a pill and/or a compressed powder, preferably an effervescent tablet, which optionally comprises an active agent.
13. The inhalation device of any of the preceding items10 to 12, wherein the bubble generating means (7) comprises a porous material and/or fabric and/or filament configured for gas to flow therethrough.
14. The inhalation device of any of the preceding items10 to 13, further comprising heating means to heat said liquid and/or said gas.
15. The inhalation device of any of the preceding items10 to 14, further comprising a guide (11) for guiding carrier gas at least partially along the surface (4) of the liquid.
16. The inhalation device of 15, the guide comprising a cap (11), the cap extending at least partially close to the surface of the liquid.
17. The inhalation device of any of the preceding items10 to 16, further comprising a terminal (12) for connection with a gas source for introducing gas into the liquid.
18. The inhalation device of any of the preceding items10 to 17, further comprising an insulating member (13) at least partially surrounding the housing (9).
19. The aerosol generated by the method of any of the preceding items1 to 9 or the inhalation device of any of the preceding claims 10 to 18 for use in a method of treating a disease, which is preferably a respiratory disease.
20. The aerosol or the inhalation device for use of items19, wherein the respiratory disease is selected from the group consisting of (i) upper respiratory diseases such as nasopharyngitis (common cold), sinusitis, pharyngitis, pharyngitis, tonsillitis, laryngitis and tracheitis, laryngitis, tracheitis, laryngotracheitis, obstructive laryngitis and epiglottitis, obstructive laryngitism, epiglottitis, bronchitis, chronic tracheitis, chronic tracheobronchitis, emphysema, asthma, status asthmaticus, bronchiectasis, allergic rhinitis, hay fever, pollinosis, chronic rhinitis, nasopharyngitis, chronic pharyngitis, chronic sinusitis, nasal polyp; (ii) influenza; (iii) pneumonia; (iv) lower respiratory infections selected from bronchitis, bronchiolitis, tracheitis, tracheobronchitis, emphysema; and (iv) other chronic obstructive pulmonary disease such as asthma, status asthmaticus, bronchiectasis.

## Claims

1. Method of generating aerosol in an inhalation device (1) for inhalation by a human or animal body, the method comprising:
Providing liquid (2) in the inhalation device (1),
Chemically generating gas bubbles (3) in the liquid (2),
Bursting of said gas bubbles (3) at the surface (4) of the liquid, thereby generating an aerosol.

2. Method of claim 1, wherein the liquid comprises a first liquid having a first density and a second liquid having a second density which is lower than the first density, wherein the first and second liquids are (substantially) immiscible.

3. Method of any of the preceding claims, wherein the liquid and/or the bubble generating means (6, 7) comprises an active agent, which preferably comprises or consists of a salt or a salt solution.

4. Method of claim 3, wherein the bubble generating means (7) further comprises a salt of carbonic acid and an organic acid.

5. Method of claim 4, wherein (i) the salt of carbonic acid is an alkali (hydrogen) carbonate and/or mixtures thereof such as sodium carbonate and potassium carbonate; sodium carbonate and/or potassium hydrogen carbonate; sodium hydrogen carbonate and/or potassium carbonate; sodium carbonate and/or sodium hydrogen carbonate; potassium carbonate and/or potassium hydrogen carbonate; and (ii) the organic acid is selected from citric acid, tartaric acid or malic acid and wherein the organic acid is preferably citric acid.

6. Method of any of the preceding claims, comprising heating of said liquid and/or said gas before generating the gas bubbles.

7. Method of any of the preceding claims, comprising chemical generating of gas bubbles by bringing the bubble generating means (6), preferably in the form of a powder, a pill, and/or compressed powder, in contact with liquid (2).

8. Method of any of the preceding claims, wherein the bubble generating means (6) includes an effervescent tablet which optionally comprises an active agent.

9. Method of any of the preceding claims, wherein the active agent is an active agent for the local or systemic treatment of a respiratory disease.

10. Inhalation device (1), preferably a disposable or single-use inhalation device, for generating aerosol and for guiding aerosol to a human or animal body for inhalation, the inhalation device comprising
an inlet opening (8) for intake of carrier gas, in particular ambient air,
a housing (9) configured to receive liquid (2) and a bubble generating means (6, 7) for generating bubbles (3) in the liquid,
the bubble generating means (6, 7) being configured to chemically generate gas bubbles (3) in the liquid (2), such that gas bubbles (3) burst at the surface of the liquid, thereby generating an aerosol, and
an outlet (10) for guiding the aerosol to the human or animal body for inhalation.

11. The inhalation device of claim 10, the inhalation device comprising the bubble generating means, the housing (9) accommodating the bubble generating means (6, 7), and/or the inhalation device comprising the liquid (2).

12. The inhalation device of claim 10 or 11, wherein the bubble generating means (6) includes a powder, a pill and/or a compressed powder, preferably an effervescent tablet, which optionally comprises an active agent.

13. The inhalation device of claim 12, wherein the bubble generating means (7) further comprises a salt of carbonic acid and an organic acid.

14. The inhalation device of claim 13, wherein (i) the salt of carbonic acid is an alkali (hydrogen) carbonate and/or mixtures thereof such as sodium carbonate and potassium carbonate; sodium carbonate and/or potassium hydrogen carbonate; sodium hydrogen carbonate and/or potassium carbonate; sodium carbonate and/or sodium hydrogen carbonate; potassium carbonate and/or potassium hydrogen carbonate; and (ii) the organic acid is selected from citric acid, tartaric acid or malic acid and wherein the organic acid is preferably citric acid.

15. The inhalation device of any of the preceding claims 10 to 14, further comprising heating means to heat said liquid and/or said gas.

16. The inhalation device of any of the preceding claims 10 to 15, further comprising a guide (11) for guiding carrier gas at least partially along the surface (4) of the liquid.

17. The inhalation device of claim 16, the guide comprising a cap (11), the cap extending at least partially close to the surface of the liquid.

18. The inhalation device of any of the preceding claims 10 to 17, further comprising a terminal (12) for connection with a gas source for introducing gas into the liquid.

19. The inhalation device of any of the preceding claims 10 to 18, further comprising an insulating member (13) at least partially surrounding the housing (9).

20. The aerosol generated by the method of any of the preceding claims 1 to 9 or the inhalation device of any of the preceding claims 10 to 19 for use in a method of treating a disease, which is preferably a respiratory disease.

21. The aerosol or the inhalation device for use of claim 20, wherein the respiratory disease is selected from the group consisting of (i) upper respiratory diseases such as nasopharyngitis (common cold), sinusitis, pharyngitis, pharyngitis, tonsillitis, laryngitis and tracheitis, laryngitis, tracheitis, laryngotracheitis, obstructive laryngitis and epiglottitis, obstructive laryngitism, epiglottitis, bronchitis, chronic tracheitis, chronic tracheobronchitis, emphysema, asthma, status asthmaticus, bronchiectasis, allergic rhinitis, hay fever, pollinosis, chronic rhinitis, nasopharyngitis, chronic pharyngitis, chronic sinusitis, nasal polyp; (ii) influenza; (iii) pneumonia; (iv) lower respiratory infections selected from bronchitis, bronchiolitis, tracheitis, tracheobronchitis, emphysema; and (iv) other chronic obstructive pulmonary disease such as asthma, status asthmaticus, bronchiectasis.
